# EUROPEAN PATENT SPECIFICATION

(11) **EP 1 917 071 B1**
(45) Date of publication and mention of the grant of the patent: **08.01.2014**
(21) Application number: 06776842.4
(22) Date of filing: 15.08.2006
(51) Int. Cl.: A61P 15/08, A23L 1/303, A23K 1/16, A61K 31/593

(54) **USE OF 25-HYDROXY VITAMIN D3 TO IMPROVE MALE ANIMAL FERTILITY**
VERWENDUNG VON 25-HYDROXY-VITAMIN-D3 ZUR VERBESSERUNG DER FERTILITÄT VON MÄNNLICHEN TIEREN
UTILISATION DE 25-HYDROXY VITAMINE D3 POUR AMÉLIORER LA FERTILITÉ D'UN ANIMAL MÂLE

(30) Priority: 18.08.2005 EP 05017949
(43) Date of publication of application: 07.05.2008
(73) Proprietor: DSM IP Assets B.V., 6411 TE Heerlen (NL)
(72) Inventor: CHUNG, Thau, Kiong, 588134 Singapore (SG); PENALBA, Francisco, Feranil, Los Banos, Laguna 4030 (PH); WEBER, Gilbert, CH-4312 Magden (CH)
(74) Representative: Schiener, Jens
(86) International application number: PCT/EP2006/008032
(87) International publication number: WO 2007/020042

(56) References cited:
- WO-A-03/059358
- WO-A2-90/06121
- AUDET I ET AL: "Effect of vitamin supplements on some aspects of performance, vitamin status, and semen quality in boars." JOURNAL OF ANIMAL SCIENCE, vol. 82, no. 2, February 2004 (2004-02), pages 626-633, XP002405449 ISSN: 0021-8812
- SINGH PAWAN ET AL: "Effect of multiple showering and vitamin supplementation on sexual behaviour, quality and freezability of buffalo bull semen" ASIAN-AUSTRALASIAN JOURNAL OF ANIMAL SCIENCES, vol. 14, no. 2, January 2001 (2001-01), pages 184-188, XP009074336 ISSN: 1011-2367
- KUPFERSCHMIED H ET AL: "[Effect of a vitamin ADE preparation on spermatic quality and offsprings of bulls used for insemination]" DEUTSCHE TIERÄRZTLICHE WOCHENSCHRIFT. MAR 1969, vol. 76, no. 6, March 1969 (1969-03), pages 142-143, XP009074349 ISSN: 0012-0847

## Description

The present invention relates to the non-therapeutical use of 25-hydroxy vitamin D₃ to improve animal fertility.

In the framework of the invention, with animals is meant animals, including mammals, examples of which include humans. Preferred examples of mammals beside humans are ruminant and non-ruminant animals, for example cows (cattle), pets as dogs and cats, horses, camels, sheep, goats and pigs.

More particularly, the invention relates to the use of 25-hydroxy vitamin D₃ for improving the quality of semen in male animals including men. In a preferred embodiment the invention relates to the use of 25-hydroxy vitamin D₃ for improving the quality of semen and/or conception rate in animals as for example cows, dogs, cats and horses, preferably pigs.

Genetically superior male animals such as boars, stallions or bulls being highly priced are not only given extraordinary management and care but also kept as long as possible to maximize their services in the herd or at an artificial insemination center. Some farms or artificial insemination centers are now accommodating their male breeding animals under optimized conditions to maintain their high level of performance, e.g. boars in a tunnel-ventilated or air conditioned pig houses. Artificial insemination is being practiced to maximize the services of the male animals and eventually transmit their superior genetics to a greater number of progenies. However, there is a tendency for semen volume, sperm motility and sperm count to gradually decline as the male animal progresses in age. As such, the rate of fertilization and conception in the female animal is gradually declining as well.

In accordance with the present invention it has been found that administering 25-hydroxy vitamin D₃ to boars improves the quality of boar semen and, further, increases the conception rate in pigs which have been fertilized by boars that were administered 25-hydroxy vitamin D₃.

Thus, in one aspect, the invention relates to the non-therapeutical use of 25-hydroxy vitamin D₃ for improving the quality of semen, for example of boar semen and conception rate in animals, for example pigs. In another aspect the invention relates to a method for improving the quality of semen and conception rate in animals which comprises administering to a male animal an effective amount of 25-hydroxy vitamin D₃.

As used herein, the term food product refers to any food or feed suitable for consumption by humans or animals. The food product may be a prepared and packaged human food (e.g., mayonnaise, salad dressing, bread, or cheese food) or an animal feed (e.g., extruded and pelleted animal feed, coarse mixed feed or pet food composition). The term food comprises also both solid and liquid food as well as drinking fluids such as drinking water. As used herein, the term foodstuff refers to any substance fit for human or animal consumption. The term dietary supplement refers to a small amount of a compound for supplementation of a human or animal diet packaged in single or multiple dose units. Dietary supplements do not generally provide significant amounts of calories but may contain other micronutrients (e.g., vitamins or minerals). The term nutritional supplement refers to a composition comprising a dietary supplement in combination with a source of calories. In some embodiments, nutritional supplements are meal replacements or supplements (e.g., nutrient or energy bars or nutrient beverages or concentrates).

For the purposes of the invention, 25-hydroxy vitamin D₃ is suitably administered as supplement to food. Animal food may be supplemented by admixing 25-hydroxy vitamin D₃, e.g., as a commercial formulation such as available under the Trademark ROVIMIX Hy-D® to regular food or by first preparing a premix of a food component and 25-hydroxy vitamin D₃ and subsequent mixing the premix with other food components. In regard to pigs, the food can be any conventional pig food. Particularly, 25-hydroxy vitamin D₃ can be added as a formulated powder to a premix containing other minerals, vitamins, amino acids and trace elements which premix is added to regular animal food and thorough mixing to achieve even distribution therein.

In the manufacture of a pig food in accordance with the invention, from about 10 µg/kg to about 100 µg/kg of 25-hydroxyvitamin D₃ are added to regular pig food. Alternatively, a food premix may be prepared on the basis of regular food components by adding active ingredients to such food components in higher concentration, e.g., in a concentration of from about 10 mg/kg to about 100 mg/kg of 25-hydroxyvitamin D₃. If one kg of such premix is added per 1000 kg of regular food this would typically meet the individual need of the animal by normal food consumption. For boars preferable dosages are in the range of and from about 50 to about 80 µg of 25-hydroxyvitamin D₃ per Kg food.

For the purposes of the invention, 25-hydroxy vitamin D₃ is suitably administered in amounts from about 0.112 µg to about 1.120 µg, especially about 0.560 µg to about 0.784 µg per kg body weight of an individual animal per day. Thus, 25-hydroxy vitamin D₃ is suitably added to the food in an amount to satisfy such dosage requirement. Typically, a boar food may contain from about 10 µg to about 100 µg, especially of from about 50 µg to about 80 µg 25-hydroxy vitamin D₃ per kg food.

The efficiency of the administration of 25-hydroxy vitamin D₃ to boars on the quality of the semen can be judged by determining the boar's total semen volume and consistency, sperm motility and sperm count as well as on the conception rates of pigs inseminated by semen from boars after administration of 25-hydroxy vitamin D₃ in comparison to the data obtained with food which does not contain 25-hydroxy vitamin D₃.

Typically for dogs 25-hydroxy vitamin D₃ is suitably administered in amounts from about 0.125 µg to about 1 µg per kg body weight of an individual animal per day and typically for horses 25-hydroxy vitamin D₃ is suitably administered in amounts from about 0.112 µg to about 1.120 µg per kg body weight of an individual animal per day.

For the consumption by human a food supplement according to the invention may be administered in the form of unit dosages, for example tablets, capsules, measured powders, or measured liquid portions, which may, if desired, be carbonated. Furthermore, the food supplements of the invention may additionally contain conventional inert and physiologically acceptable carriers, flavouring agents, colouring agents and calcium. For example a tablet may be supplemented with 25-hydroxy vitamin D₃ in an amount to satisfy a daily dosage of 25-hydroxy vitamin D₃ from about 5 to 15 µg per kg body weight, wherein the usual daily dose is 1 or 2 tablets.

The invention is further illustrated by the Examples which follow.

### Example 1: A conventional pig food comprising the components shown in Table 1 below is supplemented with 50 to 80 µg 25-hydroxy vitamin D₃ per kg of food.

**Table 1**

| Ingredient | kg/1000 kg |
|---|---|
| Corn | 314.94 |
| Corn bran | 300.00 |
| Soybean meal | 245.00 |
| Copra meal | 100.00 |
| Limestone | 14.20 |
| Mono-Dical | 15.60 |
| L-lysine.HCl | 0.47 |
| DL-methionine | 0.29 |
| NaCl | 5.00 |
| Toxin binder | 2.50 |
| Vitamin and mineral premix *) | 1.00 |
| Zinc oxide | 0.50 |
| Choline.HCl | 0.50 |
| Total | 1000.00 |
| *)Vitamin and mineral premix | |
| Vitamin A, IU/kg | 10000 |
| Vitamin D3, IU/kg | 1000 |
| Vitamin E, IU/kg | 20 |
| Vitamin K3, mg/kg | 1 |
| Vitamin B1, mg/kg | 2 |
| Vitamin B2, mg/kg | 4 |
| Vitamin B6, mg/kg | 4 |
| Vitamin B12, mg/kg | 0.01 |
| Vitamin C, mg/kg | 10 |
| Folic acid, mg/kg | 0.5 |
| Nicotinic acid, mg/kg | 20 |
| Pantothenic acid, mg/kg | 10 |
| Biotin, mg/kg | 0.08 |
| Iron, mg/kg | 100 |
| Zinc, mg/kg | 100 |
| Manganese, mg/kg | 50 |
| Copper, mg/kg | 10 |
| Iodine, mg/kg | 0.5 |

The food as specified above is supplemented with 25-hydroxy vitamin D₃ by mixing 50 to 80 mg of 25-hydroxy vitamin D₃ (4 to 6.4 g of ROVIMIX® Hy·D® 1.25% as supplied by DSM Nutritional Products, Kaiseraugst, Switzerland) together with the remaining food items. The obtained mash food, if needed, can be pelleted.

### Example 2: A food for boars is prepared as indicated in Example 1 except that about 50 to 80 mg of 25-hydroxy vitamin D₃ are added per kg of vitamin and mineral premix.

### Example 3: A premix for a boar food containing 25-hydroxy vitamin D₃ can be prepared as follows:

| Ingredients | [%] |
|---|---|
| ROVIMIX® Hy·D® 1.25% | 0.0800 |
| Vitamin A 500 | 0.8000 |
| Vitamin E 50% | 8.0000 |
| Vitamin D3 500 | 0.0800 |
| Vitamin K3 100% MSB /51% | 0.0800 |
| Vitamin B1 98% | 0.0714 |
| Vitamin B2 80% | 0.1750 |
| Vitamin B6 99% | 0.1212 |
| Vitamin B12 0.1% | 1.0000 |
| Biotin 2% | 0.2000 |
| Folic Acid 80% | 0.0227 |
| Niacin 99.5% | 0.7035 |
| Calpan 98% | 0.4082 |
| Vitamin C | 4.0000 |
| Choline chloride 60% | 12.0000 |
| Copper sulfate 25% | 12.8000 |
| Iron sulfate 30% | 10.0000 |
| Manganese oxide 62% | 1.6129 |
| Zinc oxide 76% | 5.2632 |
| Cobalt carbonate 5% | 0.0600 |
| Calcium iodate 62% | 0.0323 |
| Sodium selenite 1%/BMP | 0.8001 |
| BHT 100% | 2.0000 |
| Carrier Combination | 6.0000 |
| LACANTES S36400-Z | 10.0000 |
| Limestone | 23.6895 |

All ingredients are carefully mixed together and 0.5% (5kg/1000 kg, of food) of this premix is added to the final food.

Alternatively, 25-hydroxy vitamin D₃ can also be added in a 1% diluted premix, containing a suitable carrier. Such carrier can be wheat flour, wheat middlings, corn cobs, rice hulls, almond shells or calcium carbonate alone or in variable mixtures of several of these carriers. A typical formula is:

| Ingredients | [%] |
|---|---|
| Rice hulls | 64.20 |
| Calcium carbonate | 35.00 |
| ROVIMIX® Hy·D® 1.25% | 0.80 |

All ingredients are carefully mixed together and 0.05% (0.5 kg/1000 kg of food) of this premix is added to the final food.

### Example 4: A conventional dog food (basal diet) comprising the components shown in Table 2 and 3 below is supplemented with 10 to 80 µg 25-hydroxy vitamin D₃ (ROVIMIX® Hy·D®, 1.25%) per kg of food.

**Table 2: Nutrient composition of a basal dog diet (Mera Dog "Brocken", MERA-Tiernahrung GmbH, Marienstraße 80-84, D-47625 Kevelaer-Wetten, Germany)**

| Substance | Inclusion levels |
|---|---|
| Protein, % | 24 |
| Fat, % | 9.0 |
| Fiber, % | 3.0 |
| Ash, % | 7.0 |
| Calcium, % | 1.3 |
| Phosphor, % | 1.0 |
| Sodium, % | 0.4 |

**Table 3: Vitamin composition of a basal diet (Mera Dog "Brocken", MERA-Tiernahrung GmbH, Marienstraße 80-84, D-47625 Kevelaer-Wetten, Germany)**

| Vitamins/kg | Inclusion levels |
|---|---|
| Vitamin A, IU | 12000 |
| Vitamin D₃, IU | 1200 |
| Vitamin E, mg | 95 |
| Vitamin B₁, mg | 3.2 |
| Vitamin B₂, mg | 5.6 |
| Vitamin B₆, mg | 3.2 |
| Vitamin B₁₂, mg | 64 |
| Pantothenic acid, mg | 22 |
| Niacin, mg | 20 |
| Folic acid, mg | 0.5 |
| Biotin, mg | 200 |
| Choline chloride, mg | 1600 |

### Example 5: A conventional horse food (basal diet) comprising the components shown in Table 4 and 5 below is supplemented with 25-hydroxy vitamin D₃ (ROVIMIX® Hy·D® 1.25%) in amount to satisfy a daily dosage of 25-hydroxy vitamin D₃ from about 0.112 µg to about 1.120 µg per kg body weight.

**Table 4: Nutrient composition of a basal horse diet**

| Substance | Inclusion levels |
|---|---|
| Protein, % | 13.5 |
| Fat, % | 3.6 |
| Fiber, % | 9.7 |
| Ash, % | 5.9 |
| Calcium, % | 1.3 |
| Phosphor, % | 0.5 |
| Sodium, % | 0.3 |

**Table 5: Vitamin composition of a basal horse diet**

| Vitamins/kg | Inclusion levels |
|---|---|
| Vitamin A, IU | 36000 |
| Vitamin D₃, IU | 3000 |
| Vitamin E, mg | 1000 |
| Vitamin B₁, mg | 15 |
| Biotin, mcg | 500 |

### Example 6: A dietary supplement for human consumption in form of a tablet may comprise 25-hydroxy vitamin D₃ in a matrix component standardized for vitamin formulations. The standard matrix component may include a natural polysaccharide gum with emulsifying capacity or a mixture of different natural polysaccharide gums having emulsifying capacity without the admixture of any proteins.

| Ingredients | Amount per tablet |
|---|---|
| 25-hydroxy vitamin D₃ (crystalline) | 10 - 125 µg |
| Matrix component (standardized) | 100 - 400 mg |
| Calcium salt | 0 - 1000 mg |

The tablet may be prepared and formulated by conventional tabletting means which are well-known to one skilled in the art. Thus, for example, tablets may be prepared by mixing, pulverizing and granulating all the components of the food supplement and compressing the granulate in a tabletting machine.

## Claims

1. The use of 25-hydroxy vitamin D₃ for non-therapeutically improving the quality of semen and/or conception rate in animals, wherein 25-hydroxy vitamin D₃ is administered as a food supplement.

2. The use as in claim 1 for improving the quality of semen in pets, preferably in dogs and cats.

3. The use as in claim 1 for improving the quality of stallion semen in horses.

4. The use as in claim 1 for improving the quality of bull semen in cattle.

5. The use as in claim 1 for improving the quality of boar semen and conception rate in sows.

6. The use as in claim 1 for improving the quality of semen in human beings.

7. The use as in claim 1 wherein 25-hydroxy vitamin D₃ is added to the food in an amount required to administer from 0.1 µg to 1.2 µg, especially 0.5 µg to 0.8 µg of 25-hydroxy vitamin D₃ per kg body weight of an individual animal per day.

8. The use as in claim 7 wherein 25-hydroxy vitamin D₃ is added to boar food in an amount of 10 µg to 100 µg, especially of from 50 µg to 80 µg per kg food.

## Patentansprüche

1. Verwendung von 25-Hydroxy-Vitamin-D₃ zur nicht therapeutischen Verbesserung der Qualität des Samens und/oder der Konzeptionsrate bei Lebewesen, wobei 25-Hydroxy-Vitamin-D₃ als Nahrungsmittelergänzung verabreicht wird.

2. Verwendung nach Anspruch 1 zur Verbesserung der Qualität des Samens bei Haustieren, 10 bevorzugt Hunden und Katzen.

3. Verwendung nach Anspruch 1 zur Verbesserung der Qualität des Hengst-Samens bei Pferden.

4. Verwendung nach Anspruch 1 zur Verbesserung der Qualität des Bullensamens bei Rindern.

5. Verwendung nach Anspruch 1 zur Verbesserung der Qualität des Ebersamens und der Konzeptionsrate bei Säuen.

6. Verwendung nach Anspruch 1 zur Verbesserung der Qualität des Samens bei Menschen.

7. Verwendung nach Anspruch 1, wobei 25-Hydroxy-Vitamin-D₃ der Nahrung in einer Menge zugegeben wird, die erforderlich ist, um 0,1 µg bis 1,2 µg, speziell 0,5 µg bis 0,8 µg 25 25-Hydroxy-Vitamin-D₃ pro kg Körpergewicht eines einzelnen Lebewesens pro Tag zu verabreichen.

8. Verwendung nach Anspruch 7, wobei 25-Hydroxy-Vitamin-D₃ der Eber-Nahrung in einer Menge von 10 µg bis 100 µg, speziell 50 µg bis 80 µg pro kg Nahrung zugegeben wird.

## Revendications

1. Utilisation de 25-hydroxy vitamine D₃ pour améliorer non thérapeutiquement la qualité du sperme et/ou le taux de conception chez les animaux, dans laquelle la 25-hydroxy vitamine D₃ est administrée en tant que complément alimentaire.

2. Utilisation selon la revendication 1 pour améliorer la qualité du sperme chez les animaux domestiques, de préférence chez les chiens et les chats.

3. Utilisation selon la revendication 1 pour améliorer la qualité du sperme des étalons chez les chevaux.

4. Utilisation selon la revendication 1 pour améliorer la qualité du sperme des taureaux chez les bovins.

5. Utilisation selon la revendication 1 pour améliorer la qualité du sperme des verrats et le taux de conception des truies.

6. Utilisation selon la revendication 1 pour améliorer la qualité du sperme chez l'homme.

7. Utilisation selon la revendication 1 dans laquelle la 25-hydroxy vitamine D₃ est ajoutée à l'alimentation en une quantité nécessaire pour administrer de 0,1 µg à 1,2 µg, en particulier de 0,5 µg à 0,8 µg de 25-hydroxy vitamine D₃ par kg de poids corporel d'un animal individuel par jour.

8. Utilisation selon la revendication 7 dans laquelle la 25-hydroxy vitamine D₃ est ajoutée à l'alimentation des verrats en une quantité de 10 µg à 100 µg, en particulier de 50 µg à 80 µg par kg d'aliment.
